# EUROPEAN PATENT SPECIFICATION

(11) **EP 1 462 084 B1**
(45) Date of publication and mention of the grant of the patent: **27.05.2009**
(21) Application number: 02786019.6
(22) Date of filing: 03.12.2002
(51) Int. Cl.: A61Q 1/10, A61K 8/19

(54) **PIGMENT DISPERSION FOR COSMETIC**
PIGMENTDISPERSION FÜR KOSMETIKA
DISPERSION DE PIGMENTS POUR PRODUITS DE BEAUTE

(30) Priority: 03.12.2001 JP 2001368834; 01.10.2002 JP 2002289110
(43) Date of publication of application: 29.09.2004
(73) Proprietor: MIKUNI SHIKISO KABUSHIKI KAISHA, Himeji-shi, Hyogo 671-0234 (JP)
(72) Inventor: AOTA, Takeshi, c/o MIKUNI SHIKISO K. K., Himeji-shi, Hyogo 671-0234 (JP)
(74) Representative: Hartz, Nikolai
(86) International application number: PCT/JP2002/012638
(87) International publication number: WO 2003/047536

(56) References cited:
- JP-A- 6 336 411
- JP-A- 10 231 233
- JP-A- 63 307 809
- US-A- 5 122 418
- US-A- 5 578 311

## Description

### Technical field

This invention relates to a pigment dispersion suitably usable for black color cosmetic products and a eyeliner utilizing the same, and the like.

### Background art

Heretofore, as black pigments used in cosmetic products, carbon black, black oxide of iron and titanium black are generally known. These black pigments are used, for example, dispersed in an aqueous medium, for such an use as a pen type eyeliner, in which a cosmetic filled in a cylindrical storage space is applied by means of a brush type nib.

Among these black pigments, carbon black and titanium black are approved in some countries but not in other countries, for use as colorants in cosmetic products, thus it is difficult to use them worldwide.

On the other hand, black oxide of iron tends to agglomerate because of its magnetic property, and is difficult to be dispersed. Furthermore, it causes such a problem that it oxidizes and changes color into auburn. Particularly, in case of uses in the cosmetics, the dispersion medium is limited from the point view of safety to the skin, thus it is even more difficult in a case of an aqueous type cosmetic, to obtain a stable dispersion of black oxide of iron. As above described, it is difficult to disperse finely the pigment ingredient and a large amount of coarse particles exists. Thus, when used in a pen type cosmetic such as an eyeliner, there has been such a problem that ink is not sufficiently fed to the pen nib, that causes thin spot in writing. Also, to solve the above described problem of color change, and to improve the dispersion stability, it has been tried to blend various kinds of pigments such as black oxide of iron, red oxide of iron, Prussian blue, yellow oxide of iron, and the like, to prepare a black color cosmetic. But because the obtained dispersion is poorly dispersed, its use has been limited to be used with a pen part able to write even with a poor dispersion. Such a pen part generally has problems for instance its quality as a cosmetic apparatus is not stable, for example, ink flow rate is not stable and causes big droplets from the pen nib.

Consequently, the present inventor has conducted an intensive study to solve the above described problems, to present a pigment dispersion for cosmetic use, having an excellent dispersibility and able to be used in a pen type cosmetic. As a result, he has achieved to obtain a black color dispersion having an excellent hue and at the same time an excellent dispersion stability, by using a certain three components in combination.

US 5,122,418 describes a cosmetic composite powder that may contain black iron oxide. US 5,578,311 describes a cosmetic composition comprising a powder coated with a fluorine compound and a perfluoro organic compound.

### Description of the invention

Thus, the present invention resides in:
(1) A pigment dispersion for cosmetic products, which contains black oxide of iron, red oxide of iron and Prussian blue, and an anionic dispersing agent and a nonionic dispersing agent.
(2) A pigment dispersion for cosmetic products according to the above feature (1) or (2), **characterized in that** the viscosity is not more than 20mPa·s.
(3) A pigment dispersion for cosmetic products of the above feature (2) **characterized in that** the anionic dispersing agent contains at least one from poly-aspartic acid, poly-glutamic acid, styrene-acrylic acid copolymer, styrene-methacrylic acid copolymer and styrene-α-methyl·styrene-acrylic acid copolymer and the salts of each of these, and that the nonionic dispersing agent contains a polyoxyethylene alkylether having HLB of from 15 to 20.
(4) A pigment dispersion according to any one of the above features (1) to (4), **characterized in that** it is a water type eyeliner use.
(5) An eyeliner **characterized in that** the pigment dispersion as defined in any one of the above features (1) to (4) is filled in a pen type container.

Now, the present invention is described in detail below.

In the present invention, black oxide of iron, red oxide of iron and Prussian blue are indispensable components as pigment in the present invention. Black iron oxide alone is difficult to disperse and sufficient stabilization of dispersion is impossible. With only red oxide of iron and Prussian blue, it is difficult to obtain a desired hue. Therefore the above mentioned three components is indispensable to obtain a dispersion of black by mixed color.

A blending ratio between these three components at a weight ratio is, black oxide of iron : red oxide of iron = 3:0.5 ∼ 3:5, particularly preferably 3:1∼ 3:3, black oxide of iron : Prussian blue = 3:0.3∼ 3:5, particularly preferably 3:0.5∼ 3:3, black oxide of iron : red oxide of iron : Prussian blue = 3:1:0.5∼ 3:2:5, inter alia preferably 3:2:1∼ 3:2:3 to obtain a hue closest to black and also excellent dispersion stability. If the amount of red oxide of iron is less than the above described range, the dispersion stability is lowered. On the other hand, when the amount of red oxide of iron exceeds the above described range, the hue deviates black. Also, if the amount of Prussian blue is less than the above described range, the hue deviates black. On the other hand, if it exceeds the above described range, the hue changes and it is difficult to obtain a good black color. Furthermore dispersibility is lowered and it tends to gelate and to cause segregation. Also, to use these three pigment components to any color other than black, for example to a brown color cosmetic, is all right.

A particle size of a pigment used in the present invention is not particularly limited, but generally, the primary particle size of black oxide of iron is from 0.15 to 0.5µm, preferably from 0.15 to 0.3µm, that of red oxide of iron is generally from 0.02 to 0.7µm, preferably from 0.09 to 0.4µ m, that of Prussian blue is generally from 0.05 to 0.2µm, preferably from 0.08 to 0.15µm.

A shape of these pigments is not limited either, and those with needle shape, sphere shape, and the like, or any shape can be employed.

A concentration of the pigment in the dispersion is preferably from 1 to 40 weight percent, more preferably from 10 to 30 weight percent.

A pigment dispersion for cosmetic of the present invention is suitable for use in an aqueous type cosmetic. Here, an aqueous type means that its dispersion medium contains at least 40 weight percent of water. The dispersion medium besides water is not limited, but usually, it may contain a water soluble alcohol or a polyvalent alcohol.

A pigment dispersion of the present invention has an improved stability of the dispersed state, by using a dispersing agent. Here, as dispersing agent, using both of anionic type dispersing agent and nonionic type dispersing agent is especially recommended.

With the above described pigment formula, if only an anionic type dispersing agent is used, the stability of the system is low and, it may cause viscosity increase and gelation and tends to cause problem such as bad writing feeling of ink due to thixotropy. If only a nonionic type dispersing agent is used, dispersibility is not sufficient and it may cause such problems as color split of pigment, and difference of density between nib up state and nib down state. By using both of anionic type dispersing agent and nonionic type dispersing agent, a cosmetic showing no difference of density between nib up state and nib down state can be obtained.

Examples of an anionic type dispersing agent are, one or equal or more than two from the following: polyaspartic acid, polyglutamic acid, styrene/acrilic acid copolymer, styrene/metacrilic acid copolymer and styrene/α-methyl styrene/acrilic acid copolymer, and salts of these. Namely, as anionic type dispersing agent, it is sufficient to contain at least one of the following: polyaspartic acid, polyglutamic acid, styrene/acrilic acid copolymer, styrene/metacrilic acid copolymer and styrene/α-methyl styrene/acrilic acid copolymer, or at least one of their salts.

Their salts are not limited, but for example, alkali salts such as sodium salts, potassium salts, lithium salts; ammonium salts; and alkanol salts such as mono-, di-, tri- ethanol amine, triisopropanol amine, may be used.

Among the above described anionic type dispersing agent, especially, to use at least one from the following: polyaspartic acid, polyglutamic acid, styrene/acrilic acid copolymer, styrene/metacrilic acid copolymer and styrene/α-methyl styrene/acrilic acid copolymer and their salts, is preferred as dispersing stability is especially excellent.

With respect to the properties such as dispersibility of the pigment dispersion and hue and stability thereof, the molecular weight of the anionic type dispersing agent is preferably from 1000 to 20000, more preferably from 2000 to 13000.

Especially, using polyaspartic acid and/or its salts as anionic type dispersing agent, compared to when other anionic type dispersing agent is used, the viscosity of the dispersion system drops strikingly, therefore there is such an advantage that a good dispersion is possible even with high concentration of pigment, therefore color density of ink of the writing line increases.

As nonionic type dispersing agent, there are:
Polyoxyethylene type nonionic surface agent such as: polyoxyethylene laurylether,polyoxyethylene oleylether, polyoxyethylen cetylether, polyoxyethylene stearylether, polyoxyethylene octylether, polyoxyethylene stearylether, polyoxyethylene octylether, polyoxyethylene nonylphenylether, poloxyethylene 2-octyldodecylether, polyoxyethylene isocetylether, polyoxyethylene isostearylether, polyoxyethylene cetyl/oleylether, and the like. Among them, especially, using polyoxyethylene alkylether having HLB 15-20, is preferable, as dispersion stability is excellent.

Of course, a combination of 2 or more kinds of anionic type dispersing agent, a combination of 2 or more kinds of nonionic type dispersing agent may be used.

The amount of these dispersing agent is, to 100 parts by weight of total amount of pigment, 1-60 parts by weight of anionic type dispersing agent, especially 5-35 parts by weight, is preferable. To 100 parts by weight of total amount of pigment, 1-60 parts by weight of nonionic type dispersing agent, especially 5-35 parts by weight, is preferable.

The blending ratio between anionic type dispersing agent and nonionic type dispersing agent is, by weight ratio, 100:50 ∼ 100:200 especially 100:70 ∼100:180.

The pigment dispersion of the present invention may include further additives such as humectants, preservatives, pH -adjusters. As humectants, there are for example, ethylene glycol, diethylene glycol, triethylene glycol, propylene glycol, dipropylene glycol, polyprolylene glycol, 1,3-butylene glycol, isoprene glycol, polyethylene glycol, 2-methyl-2,4-pentanediol, glycerin, concentrated glycerin, diglycerin, polyglycerin, sorbitol, sorbitol solution, maltitol, maltitol solution, xylitol.

As preservatives, there are for example, chlorobutanol, chlorocresol, parachloromethaxylenol, cresol, triclosan, trichlorocarbanilide, isopropylmethylphenol, benzethonium chloride, benzethonium chloride solution, cetylpyridinium chloride, thianthol, phenol, sodium paraphenolsulfonate (dihydrate), zind paraphenol sulfonate, resorcin, Photosensitizing Dye No.101, Photosensitizing Dye No.201, Photosensitizing Dye No.301, Photosensitizing Dye No.401, hinokitiol, 1-menthol, dl-menthol, d-camphor, dl-camphor, benzoic acid, sodium benzoate, parahydroxybenzoate type, sorbic acid, potassium sorbate, dehydroacetic acid, sodium dehydroacetate, salicylic acid, sodium salicylate, methyl salicylate, phenyl salicylate, and the like.

As pH-adjusters, there are for example, ethanolamine, diethanolamine, triethanolamine, isopropanolamine, diisopropanolamine, triisopropanolamine, 2-amino-2-methyl-1-propanol, 2-amino-2-methyl-1,3-propanediol, tetrahydro-1,4-oxazine, sodium hydroxide, magnesium hydroxide, potassium hydroxide, strong ammonia solution.

In a pigment dispersion of the present invention for cosmetic products, besides the above mentioned ingredients, the ingredients that may be generally included in an aqueous cosmetic product, for example, thickener, chilating agents, antifoaming agents, may be added appropriately as far as the effect of the present invention is not undermined.

The pigment besides the three essential component above described, for instance, for the object of obtaining a matt impression, the addition of body color such as kaolin, barium sulfate, barium titanium oxide, talc, sericite, mica is all right. From the point of view to obtain a desired black hue, it is recommended to keep to at most 20 weight percent, preferably at most 10 weight percent, based on total amount of pigment.

A pigment dispersion of the present invention may include a polymer emulsion. As polymer emulsion, typically, there are such water-soluble polymer emulsion as: acrilic acid resin type, styrene/acrilic acid resin type, vinyl acetate type, styrene/butadiene resin type, polyurethane type, olefine resin type, alkyd resin type, etc. As a thickener, there are: methylcellulose, carboxymethylcellulose, hydroxymethylcellulose, hydroxypropylcellulose, polyvinyl alcohol, polyvinylpyrrolidone, carboxyvinyl polymer, sodium polyacrylate, polyethylene glycol, ethylene oxide-propylene oxide block copolymer, acacia? (gum Arabic) Sodium alginate, xanthan gum, casein, guargum, locust bean gum, bentonite type thickener.

A pigment dispersion of the present invention may be obtained by blending each of these components. The blending method is not particularly limited, and the blending order is not limited either. Known methods may be appropriately utilized. Plural pigments may be blended beforehand and then blended with dispersing medium, dispersant, and the like, or each pigment component may be beforehand prepared as a dispersion, then mixed with each other. Also, the mixing apparatus is not particularly limited, and may be dispersed by appropriately utilizing known dispersing machines such as media mills such as ball mill, sand mill, roll mill; homomixer; attrition mill; or the like.

A pigment dispersion of the present invention as above described can express black color of excellent hue, and there is no color split, and also coarse particles of pigment is restrained and the dispersed diameter is near of primary particle size, and stability during the passage of time is also good and has an excellent dispersibility. Therefore, it is favorably utilizable especially in a pen type cosmetic product.

A viscosity of a pigment dispersion of the present invention is not particularly limited, but as it has good dispersibility, it may be a very low viscosity dispersion, for example its viscosity may be restrained to not more than 20mPa· s, even not less than 10mPa·s. If the viscosity is more than 20mPa·s, ink tends not to be delivered easily when filled in a pen type container, so smooth writing feeling is degraded. So, a pigment dispersion of the present invention, when filled in a pen type container and used as a cosmetic product such as eyeliner, it has such advantages as it is easily dischargeable from the pen, and writing feeling is smooth. Also, its handling and blending with other ingredients of the cosmetic products are easy. To obtain a higher viscosity, it may be adjusted by addition of such components as a thickner.

Besides, a pigment dispersion of the present invention may be one containing no organic solvent, therefore there is an advantage such that a product such as an aqueous eyeliner wherein no worry of harm to living body, inflammability, solvent smell, may be provided.

### Examples

The present invention is further illustrated by the following examples of the invention. The terms "part" and "%" are based on weight unless otherwise specified.

### (viscosity measurement method)

In each example and comparative example, the viscosity is measured by the following method.

A sample is homogeneously stirred, then 1.2cc is taken by means of a syringe, and after that, at 25C, by means of a E-type viscosity meter (ELD type), the viscosity after 1 minute at 50rpm is measured. The measurement rotor is selected appropriately according to the viscosity of the sample.

### Example 1

The following ingredients were blended and mixed by a media mill, and a black pigment dispersion for cosmetic product were obtained.

### [Blending formula]

| | |
|---|---|
| black oxide of iron | 7.5 parts |
| red oxide of iron | 5.0 parts |
| Prussian blue | 4.0 parts |
| styrene/α-methylstyrene/acrilic acid copolymer | 6.3 parts |
| polyoxyethylene oleylether (20E.O., HLB 17.0) | 4.0 parts |
| 1,3-butylene glycol | 3.5 parts |
| 2-phenoxyethanol | 0.5 parts |
| methyl parahydroxybenzoate | 0.2 parts |
| propyl parahydroxybenzoate | 0.1 parts |
| 2-amino-2-methyl-1-propanol | 0.1 parts |
| disodium edetate | 0.1 parts |
| strong ammonia solution | 1.6 parts |
| purified water | 67.1 parts |

The black color pigment dispersion for cosmetics above described had a viscosity of 6.7 mPa·s.

The black color pigment dispersion for cosmetics above described was filled in a brush pen type container, and the writing was conducted. The obtained writing line visually observed had a good blueish black hue.

Also, after allowing to stand the above described dispersion in this container, both at room temperature and at 50°C, and both nib up and nib down state, the ink discharge was good and no difference in density of writing or hue were observed.

### Example 2

The following ingredients were blended and mixed by a media mill, and a black pigment dispersion for cosmetic product were obtained.

### [Blending formula]

| | |
|---|---|
| black oxide of iron | 10.0 parts |
| red oxide of iron | 6.8 parts |
| Prussian blue | 7.2 parts |
| sodium polyaspartate | 1.5 parts |
| polyoxyethylene laurylether (21E.O., HLB 19.0) | 2.4 parts |
| 1,3-butylene glycol | 4.0 parts |
| 2-phenoxyethanol | 0.8 parts |
| methyl parahydroxybenzoate | 0.3 parts |
| propyl parahydroxybenzoate | 0.1 parts |
| 2-amino-2-methyl-1-propanol | 0.2 parts |
| disodium edetate | 0.1 parts |
| purified water | 57.6 parts |

The black color pigment dispersion for cosmetics above described had a viscosity of 9.0 mPa·s.

The black color pigment dispersion for cosmetics above described was filled in a brush pen type container, and the writing was conducted. The obtained writing line visually observed had a good reddish black hue.

Also, after allowing to stand the above described dispersion in this container, both at room temperature and at 50°C, and both nib up and nib down state, the ink discharge was good and no difference in density of writing or hue were observed.

### Example 3

The following components were blended and mixed by a media mill, and a black pigment dispersion for cosmetic product were obtained.

### [Blending formula]

| | |
|---|---|
| black oxide of iron | 8.8 parts |
| red oxide of iron | 12.0 parts |
| Prussian blue | 2.4 parts |
| sodium polyaspartate | 1.2 parts |
| polyoxyethylene laurylether (21E.O., HLB 19.0) | 2.0 parts |
| alkyl acrylate copolymer emulsion | 9.0 parts |
| 1,3-butylene glycol | 4.0 parts |
| 2-phenoxyethanol | 0.8 parts |
| methyl parahydroxybenzoate | 0.3 parts |
| propyl parahydroxybenzoate | 0.1 parts |
| 2-amino-2-methyl-1-propanol | 0.2 parts |
| disodium edetate | 0.1 parts |
| purified water | 59.1 parts |

The umber color pigment dispersion for cosmetics above described had a viscosity of 7.7 mPa·s.

The umber color pigment dispersion for cosmetics above described was filled in a brush pen type container, and the writing was conducted. The obtained writing line visually observed had a good umber hue.

Also, after allowing to stand the above described dispersion in this container, both at room temperature and at 50°C, and both nib up and nib down state, the ink discharge was good and no difference in density of writing or hue were observed.

### Comparative example 1

### [Blending formula]

| | |
|---|---|
| black oxide of iron | 20.0 parts |
| red oxide of iron | 5.0 parts |
| styrene/α-methylstyrene/acrilic acid copolymer | 5.0 parts |
| 1,3-butylene glycol | 3.5 parts |
| 2-phenoxyethanol | 0.5 parts |
| methyl parahydroxybenzoate | 0.2 parts |
| propyl parahydroxybenzoate | 0.1 parts |
| disodium edetate | 0.1 parts |
| 2-amino-2-methyl-1-propanol | 0.2 parts |
| strong ammonia solution | 1.3 parts |
| purified water | 64.1 parts |

The black color pigment dispersion for cosmetics above described had a viscosity of 6.0 mPa·s.
The black color pigment dispersion for cosmetics above described was filled in a brush pen type container, and the writing was conducted. The obtained writing line visually observed and its hue was umber.

Also, after allowing to stand the above described dispersion in this container, both at room temperature and at 50°C, and both nib up and nib down state, the ink discharge was good and no difference in density of writing or hue were observed.

### Comparative example 2

### [Blending formula]

| | |
|---|---|
| black oxide of iron | 24.0 parts |
| Prussian blue | 1.0 part |
| styrene/α-methylstyrene/acrilic acid copolymer | 6.3 parts |
| polyoxyethylene oleylether (20E.O., HLB 17.0) | 4.0 parts |
| 1,3-butylene glycol | 3.5 parts |
| 2-phenoxyethanol | 0.5 parts |
| methyl parahydroxybenzoate | 0.2 parts |
| propyl parahydroxybenzoate | 0.1 parts |
| disodium edetate | 0.1 parts |
| 2-amino-2-methyl-1-propanol | 0.2 parts |
| strong ammonia solution | 1.6 parts |
| purified water | 58.5 parts |

The black color pigment dispersion for cosmetics above described had a viscosity of 7.0 mPa·s.

The black color pigment dispersion for cosmetics above described was filled in a brush pen type container, and the writing was conducted. The obtained writing line visually observed had a blueish black hue.

Also, after allowing to stand the above described dispersion in this container, both at room temperature and at 50°C, and both nib up and nib down state, the ink discharge was bad and big differences in density of writing and hue were observed.

### Comparative example 3

### [Blending formula]

| | |
|---|---|
| black oxide of iron | 25.0 parts |
| styrene/α-methylstyrene/acrilic acid copolymer | 6.3 parts |
| 1,3-butylene glycol | 3.5 parts |
| 2-phenoxyethanol | 0.5 parts |
| methyl parahydroxybenzoate | 0.2 parts |
| propyl parahydroxybenzoate | 0.1 parts |
| disodium edetate | 0.1 parts |
| 2-amino-2-methyl-1-propanol | 0.2 parts |
| strong ammonia solution | 1.6 parts |
| purified water | 62.5 parts |

The black color pigment dispersion for cosmetics above described had a viscosity of 7.0 mPa·s.

The black color pigment dispersion for cosmetics above described was filled in a brush pen type container, and the writing was conducted. The obtained writing line visually observed had a reddish black hue. Also, the ink supply to the nib was unsatisfactory and the obtained writing line had thin spots.

### Comparative example 4

### [Blending formula]

| | |
|---|---|
| black oxide of iron | 14.0 parts |
| Prussian blue | 7.0 parts |
| styrene/α-methylstyrene/acrilic acid copolymer | 2.5 parts |
| polyoxyethylene oleylether (20E.O., HLB 17.0) | 2.0 parts |
| 1,3-butylene glycol | 3.5 parts |
| 2-phenoxyethanol | 0.5 parts |
| methyl parahydroxybenzoate | 0.2 parts |
| propyl parahydroxybenzoate | 0.1 parts |
| disodium edetate | 0.1 parts |
| 2-amino-2-methyl-1-propanol | 0.2 parts |
| strong ammonia solution | 0.6 parts |
| purified water | 69.3 parts |

The black color pigment dispersion for cosmetics above described had a viscosity of 4.0 mPa·s.

The black color pigment dispersion for cosmetics above described was filled in a brush pen type container, and the writing was conducted. The obtained writing line visually observed had a blueish black hue.

Also, after allowing to stand the above described dispersion in this container, both at room temperature and at 50°C, big differences of density of writing and hue between nib up and nib down state were observed.

### Industrial Applicability

The present invention provides a pigment dispersion exhibiting a good black hue, and also an excellent dispersion stability and excellent as a pen type cosmetic when filled in a pen type container.

## Claims

1. A pigment dispersion for cosmetic products, which contains black oxide of iron, red oxide of iron and Prussian blue, said pigment dispersion containing an anionic dispersing agent and a nonionic dispersing agent.

2. A pigment dispersion for cosmetic products according to claim 1, **characterized in that** the viscosity is not more than 20mPa·s.

3. A pigment dispersion for cosmetic products of claim 1 **characterized in that** the anionic dispersing agent contains at least one from poly-aspartic acid, poly-glutamic acid, styrene-acrylic acid copolymer, styrene-methacrylic acid copolymer and styrene-α-methyl·styrene-acrylic acid copolymer and the salts of each of these, and that the nonionic dispersing agent contains a polyoxyethylene alkylether having HLB of from 15 to 20.

4. A pigment dispersion according to any one of Claims 1 to 3, **characterized in that** it is a water type eyeliner use.

5. An eyeliner **characterized in that** the pigment dispersion as defined in any one of Claims 1 to 3 is filled in a pen type container.

## Patentansprüche

1. Pigmentdispersion für kosmetische Produkte, die Eisenoxidschwarz, Eisenoxidrot und Berliner Blau enthält, wobei die Pigmentdispersion ein anionisches Dispergiermittel und ein nichtionisches Dispergiermittel enthält.

2. Die Pigmentdispersion für kosmetische Produkte gemäß Anspruch 1, **dadurch** charakterisiert, dass die Viskosität nicht größer als 20 mPa·s ist.

3. Die Pigmentdispersion für kosmetische Produkte gemäß Anspruch 1, **dadurch** charakterisiert, dass das anionische Dispergiermittel mindestens eines der folgenden enthält: Polyasparaginsäure, Polyglutaminsäure, Styrol-Acrylsäure-Copolymer, Styrol-Methacrylsäure-Copolymer und Styrol-α-Methylstyrol-Acrylsäure-Copolymer und deren Salze, und dass das nichtionische Dispergiermittel einen Polyoxyethylenalkylether mit einem HLB-Wert von 15 bis 20 enthält.

4. Die Pigmentdispersion für kosmetische Produkte gemäß einem der Ansprüche 1 bis 3, **dadurch** charakterisiert, dass sie ein Eyeliner vom Wassertyp ist.

5. Eyeliner **dadurch gekennzeichnet, dass** die Pigmentdispersion wie in einem der Ansprüche 1 bis 3 definiert in ein stiftartiges Behältnis eingefüllt ist.

## Revendications

1. Dispersion de pigments pour produits cosmétiques, qui contient de l'oxyde de fer noir, de l'oxyde de fer rouge et du bleu de Prusse, ladite dispersion de pigments contenant un agent de dispersion anionique et un agent de dispersion non ionique.

2. Dispersion de pigments pour produits cosmétiques selon la revendication 1, **caractérisée en ce que** la viscosité n'est pas supérieure à 20 mPa.s.

3. Dispersion de pigments pour produits cosmétiques selon la revendication 1, **caractérisée en ce que** l'agent de dispersion anionique contient au moins l'un parmi le poly(acide aspartique), le poly(acide glutamique), un copolymère de styrène-acide acrylique, un copolymère de styrène-acide méthacrylique, un copolymère de styrène-α-méthyl styrène-acide acrylique et les sels de chacun de ceux-ci, et **en ce que** l'agent de dispersion non ionique contient un polyoxyéthylène alkyléther ayant une valeur HLB de 15 à 20.

4. Dispersion de pigments selon l'une quelconque des revendications 1 à 3, **caractérisée en ce qu'**il s'agit d'une utilisation pour un produit à maquiller les yeux de type aqueuse.

5. Produit à maquiller les yeux **caractérisé en ce que** la dispersion de pigments telle que définie dans l'une quelconque des revendications 1 à 3 est introduite dans un conteneur de type stylo.
